# EUROPEAN PATENT APPLICATION

(11) **EP 3 656 326 A1**
(43) Date of publication of application: **27.05.2020**
(21) Application number: 17918195.3
(22) Date of filing: 17.08.2017
(51) Int. Cl.: A61B 18/14

(54) **RADIOFREQUENCY ABLATION CATHETER AND SYSTEM**

(30) Priority: 20.07.2017 CN 201710597866
(71) Applicant: Changzhou Lunghealth Medtech Company Limited, Jiangsu 213145 (CN)
(72) Inventor: CHENG, Jian, Jiangsu 213145 (CN); LIU, Hongyi, Jiangsu 213145 (CN); MA, Jiajun, Jiangsu 213145 (CN)
(74) Representative: Renaudo, Adrien Hanouar
(86) International application number: PCT/CN2017/097887
(87) International publication number: WO 2019/015003

(57) **Abstract**

Embodiments of the present application provide a radiofrequency ablation catheter and a radiofrequency ablation system. The radiofrequency ablation catheter includes: a catheter body; a ring electrode, annularly provided at a head of the catheter body; a location sensor, provided in the catheter body; and an ablation needle, provided in the catheter body and capable of moving forward or backward. The ablation needle, when applied, pushes out from the head of the catheter body and penetrates into a lesion tissue. The embodiments of the invention enable an accurate location of the lesion position and the puncture into lesion for performing radiofrequency ablation therapy, so as to achieve the targeted precise ablation therapy and reduce the damage to normal tissues.

## Description

### CROSS-REFERENCE TO RELATED APPLICATIONS

This application makes a reference to the Chinese Patent Application No. 2017105978669, entitled "Radiofrequency Ablation Catheter and System" and filed on July 20, 2017, which is incorporated herein by reference in its entirety.

### Technical Field

The present application relates to the field of medical instruments, and in particular to a radiofrequency ablation catheter and a radiofrequency ablation system.

### Background Art

Radiofrequency ablation is a minimally invasive in-situ treatment technique for tumors, which converts a radiofrequency signal into a temperature field, for treating human tissues through thermal effects. When a radiofrequency ablation treatment is applied, an electrode needle is directly inserted into a lesion tissue, a high-frequency alternating current at distal end of the electrode needle is injected into the lesion tissue and ions in the lesion tissue change along with the change of the current direction, such that the heat is generated, and a temperature of the lesion local tissue raises. When the temperature of the tissue exceeds 60°C, the cells are killed, finally coagulating and inactivating tumor tissues.

A conventional radiofrequency thermal ablation is performed by directly inserting an ablation needle into a tumor through percutaneous puncture under the guidance of an image technology such as ultrasonography or computed tomography (CT). For lung tumors, an ablation electrode needle is used to access to inner tumor via needle-puncture in the percutaneous procedure, which may cause a risk of pneumothorax. Studies have shown that pulmonary percutaneous insertion of the electrode needle into tumors has a 20%-40% incidence of pneumothorax, and pneumothorax is a serious adverse reaction which may be fatal.

A safer way is to use an ablation catheter with electrodes to access a lung tumor position through a natural airway, and then apply a radiofrequency thermal ablation to the lung tumor through the ablation catheter, thereby reducing the risk of adverse reactions. However, most of the lung lesions are located on one side of a bronchus. With simple cylindrical ablation electrodes, the ablation catheter can approach the lesions through the bronchus but cannot penetrate into the interior of the tumor, and can only be close to the tumor position. Only a part of the radiofrequency field generated by the ablation catheter acts on the tumor, and other parts of the radiofrequency field act on normal tissues, causing the normal tissues to be affected and inactivated, and the area of ablation inactivation is too large.

Therefore, there is an urgent need for an ablation catheter and system that can enter the lung through natural airways and can accurately locate the lesion and penetrate into the lesion to perform radiofrequency ablation therapy.

### Summary of the Invention

Various aspects of the invention provide a radiofrequency ablation catheter and a radiofrequency ablation system, which are used to accurately locate the lesion position and puncture into the lesion to perform radiofrequency ablation therapy, so as to achieve targeted precise ablation therapy and reduce the damage to normal tissues.

Embodiments of the application provide a radiofrequency ablation catheter, which includes:
a catheter body;
a ring electrode, annularly provided at a head of the catheter body;
a location sensor, provided in the catheter body; and
an ablation needle, provided in the catheter body and capable of moving forward or backward, wherein the ablation needle, when applied, pushes out from the head of the catheter body and penetrates into a lesion tissue.

In an alternative embodiment, the location sensor is configured to output, according to a location magnetic field in a space where the location sensor is located, a current signal to a control system external to the radiofrequency ablation catheter, such that the control system determines a position of the radiofrequency ablation catheter and controls the ablation needle and the ring electrode to perform radiofrequency ablation.

In an alternative embodiment, the catheter body has a multi-lumen structure; the location sensor is set in one of lumens and the ablation needle is set in others of the lumens.

In an alternative embodiment, the same number of ablation needles are provided in each of the other lumens.

In an alternative embodiment, the ablation needle is a straight needle which is straight after pushing out of the catheter body; or,
the ablation needle is a curved needle which is curving, after pushing out of the catheter body, at a set angle with respect to the catheter body.

In an alternative embodiment, a tail end of the ablation needle is connected to one end of a push-pull/torque transmission mechanism, and another end of the push-pull/torque transmission mechanism is connected to a push-pull handle.

In an alternative embodiment, the ring electrode is provided with a temperature sensor therein.

In an alternative embodiment, electrodes of at least two polarities are present in the ablation needle and the ring electrode.

In an alternative embodiment, the polarity of the ablation needle is opposite to the polarity of the ring electrode.

In an alternative embodiment, a portion, other than the needle tip, of the ablation needle is provided with an insulating layer.

The embodiments of the application further provide a radiofrequency ablation system, which includes: the radiofrequency ablation catheter provided in the above embodiments, a control system for controlling operations of the radiofrequency ablation catheter, and a connector for connecting the radiofrequency ablation catheter with the control system.

In the embodiments of the application, the radiofrequency ablation catheter is further provided with an ablation needle in addition to the ring electrode, and the ablation needle which is capable of moving forward or backward and pushed out of the head of the catheter body and penetrated into the lesion tissue; in addition, a location sensor is provided in the catheter body, and navigation can be performed with the location sensor, such that the radiofrequency ablation catheter may be accurately located to the lesion position, and an ablation needle can further accurately penetrate into the lesion tissue to perform radio frequency ablation therapy, so as to achieve targeted precise ablation therapy and reduce the damage to normal tissues.

### Brief Description of the Drawings

Drawings described herein are used for providing further understandings of the present application and constitute a part of the present application. Illustrative embodiments and descriptions of the present application are used for explaining the present application, and do not constitute an improper limitation to the present application. In the drawings:
Drawings described herein are used for providing further understandings of the present application and constitute a part of the present application. Illustrative embodiments and descriptions of the present application are used for explaining the present application, and do not constitute an improper limitation to the present application. In the drawings:
FIG. 1a is a schematic diagram of a structure of a radiofrequency ablation catheter in accordance with an embodiment of the present application;
FIG. 1b is a schematic diagram of an ablation needle of a radiofrequency ablation catheter, which pushes out of the head of a catheter body in accordance with an embodiment of the present application;
FIG. 2 is a stereoscopic diagram of a radiofrequency ablation catheter having a single-lumen structure in accordance with another embodiment of the present application;
FIG. 3 is a schematic diagram of a radiofrequency ablation catheter having a multi-lumen structure in accordance with another embodiment of the present application;
FIG. 4 is a schematic diagram of a radiofrequency ablation catheter with a single straight needle based on a single-lumen structure in accordance with yet another embodiment of the present application;
FIG. 5 is a schematic diagram of a radiofrequency ablation catheter having a single curved needle based on a single-lumen structure in accordance with yet another embodiment of the present application;
FIG. 6 is a schematic diagram of a radiofrequency ablation catheter having a plurality of curved needles based on a single-lumen structure in accordance with yet another embodiment of the present application;
FIG. 7 is a schematic diagram of a radiofrequency ablation catheter having a plurality of curved needles based on a multi-lumen structure in accordance with yet another embodiment of the present application; and
FIG. 8 is a schematic diagram of a structure of a radiofrequency ablation system in accordance with yet another embodiment of the present application.

### Detailed Description of the Invention

The objectives, technical solution and advantages of the present application become more apparent from the following detailed and complete description of the technical solution of the present application taken in conjunction with the specific embodiments and the accompanying drawings of the present application. It is apparent that the embodiments described herein are merely a part of embodiments of the present application and not all of the embodiments. Based on the embodiments of the present application, all other embodiments obtained by a person of ordinary skill in the art without involving any inventive effort are within the scope of protection of the present application.

Aiming at the problems in the existing radiofrequency ablation, the embodiments of the present application provide a novel radiofrequency ablation catheter. The radiofrequency ablation catheter is further provided with an ablation needle in addition to the ring electrode, and the ablation needle which is capable of moving forward or backward and pushed out of the head of the catheter body and penetrated into the lesion tissue; in addition, a location sensor is provided in the catheter body, and navigation can be performed with the location sensor, such that the radiofrequency ablation catheter may be accurately located to the lesion position, and an ablation needle can further accurately penetrate into the lesion tissue to perform radio frequency ablation therapy, so as to achieve targeted precise ablation therapy and reduce the damage to normal tissues.

The radiofrequency ablation catheter provided in the embodiments of the present application includes a ablation needle that can move forward or forward and a location sensor, and the structure of this radiofrequency ablation catheter is inevitably different from that of a traditional radiofrequency ablation catheter; in addition, considering that radiofrequency ablation catheter is a relatively sophisticated medical instrument, it is not easy to add new devices or functions, and often requires repeated experiments to realize a more reasonable implementation structure. The structure of the radiofrequency ablation catheter provided in the embodiments of the present application will be described in detail with reference to the accompanying drawings.

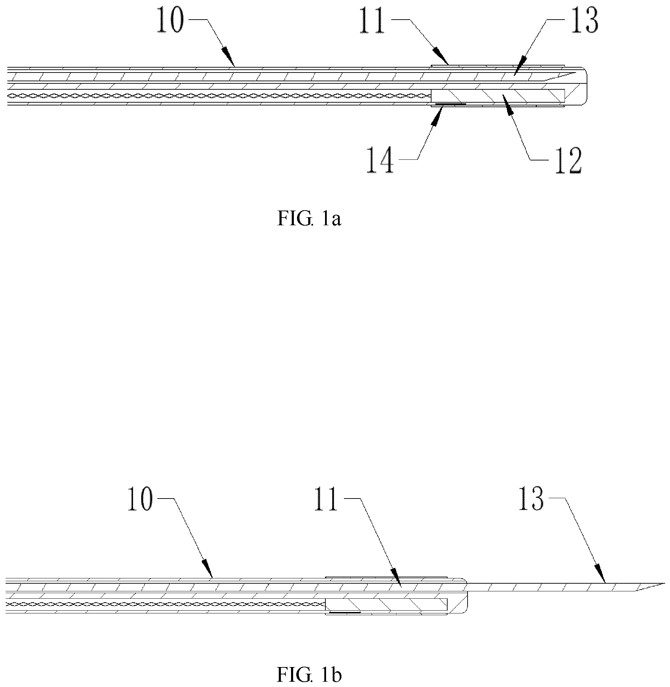

FIG. 1a is a schematic diagram of a structure of a radiofrequency ablation catheter in accordance with an embodiment of the present application. As shown in FIG. 1a, the radiofrequency ablation catheter includes: a catheter body 10, a ring electrode 11 annularly provided at a head of the catheter body 10, a location sensor 12 provided in the catheter body 10, and an ablation needle 13 provided in the catheter body 10 and capable of moving forward or backward.

Alternatively, the ring electrode 11 may be fixed to the head of the catheter body 10 by various means such as hot melting, adhesive bonding, swaging, microfluidic processing techniques, etc. Alternatively, the shape of the ring electrode 11 is not limited in the embodiments. For example, the ring electrode 11 may have a cylindrical shape, a sheet shape, a strip shape, a curved wire shape, or a disc shape.

The ablation needle 13 has a certain elasticity and may be electrically conductive. Alternatively, the ablation needle 13 and the ring electrode 11 may be made of a metallic material, such as copper, stainless steel, platinum-iridium alloy, etc., or may be made of other conductive materials. The ablation needle 13 and the ring electrode 11 cooperate with each other and are mainly used to perform ablation therapy on lesion tissues. The ablation needle 13, when applied, may push out from the head of catheter body 10 and penetrate into the lesion tissues. As shown in FIG. 1b, the ablation needle 13 pushes out of the head of the catheter body 10. The ablation needle 13 and the ring electrode 11 both generate a radiofrequency current, and the radiofrequency current is injected into the lesion tissue, such that ions in the lesion tissue change along with the change of the current direction. The temperature of the lesion tissue raises, and when the temperature exceeds a certain temperature (generally 60°C), the lesion tissue can be killed, thereby finally coagulating and inactivating tumor tissue to achieve a curative purpose.

In the embodiments, the location sensor 12 is configured to output a current signal to a control system external to the radiofrequency ablation catheter, according to a location magnetic field in a space in which the location sensor 12 is located, such that the control system determines a position of the radiofrequency ablation catheter. Further, the location sensor 12 is also configured to output a current signal to a control system external to the radiofrequency ablation catheter, according to the location magnetic field in the space in which the location sensor 12 is located, such that the control system controls the ablation needle 13 and the ring electrode 11 to perform radiofrequency ablation.

Alternatively, the location sensor 12 in the embodiments employs an electromagnetic navigation principle, which is different from the general magnetic navigation principle. The magnetic navigation principle is mainly as follows: permanent magnets in the ablation catheter are attracted or repelled by means of an external magnetic field to affect the moving direction of the ablation catheter. The working principle of the location sensor 12 in the embodiments is mainly as follows: a current signal is generated in response to a location magnetic field in a space where the location sensor 12 is located, and is output to a control system external to the radiofrequency ablation catheter, such that the control system determines the position of the radiofrequency ablation catheter on the one hand, and the control system determines the positions of the ablation needle 13 and the ring electrode 11 relative to a lesion area on the other hand.

The location sensor 12 is electrically connected to the control system by wires. The control system includes a magnetic field generator configured to generate a magnetic field in a certain location space; the location sensor 12 itself is not magnetic, and a coil in the location sensor 12 is used to sense the location magnetic field generated by the magnetic field generator. The magnetic field generator generates the location magnetic field in a certain location space so as to ensure that the magnetic characteristic of each point in the location space is unique. The coil in the location sensor 12 generate a current signal in a changing magnetic field, the current signal is conducted to the control system by the wires of the location sensor 12, and the control system converts and analyzes the current signal transmitted by the location sensor 12 to determine a precise position of the radiofrequency ablation catheter and positions of the ablation needle 13 and the ring electrode 11 in the radiofrequency ablation catheter relative to the lesion area.

Alternatively, the above-mentioned control system further includes: a radiofrequency ablation generator for controlling the ablation needle 13 and the ring electrode 11 to generate a radiofrequency current. For example, the intensity, time and etc. of the radiofrequency current generated by the ablation needle 13 and the ring electrode 11 may be controlled. The ablation needle 13 and the ring electrode 11 may generate a radiofrequency current under the control of the radiofrequency ablation generator.

The working principle of the radiofrequency ablation catheter provided in the embodiments is shown as follows.

Under the guidance of the location sensor 12, a navigation path which reaches the area where the lesion tissue is located through natural airways is planned on CT images of a pathological organ or a three-dimensional model reconstructed by the CT data; the radiofrequency ablation catheter is guided to the area where the lesion tissue is located through the natural airways according to the navigation path; the positions of the ablation needle 13 and the ring electrode 11, relative to the lesion tissue, in the radiofrequency ablation catheter are determined according to the location sensor 12; the ablation needle 13 is penetrated into the lesion tissue; the ablation needle 13 and the ring electrode 11 are powered; and then the ablation needle 13 and the ring electrode 11 are controlled to generate a radiofrequency current which is injected into the lesion tissue for ablation. In the embodiments, the radiofrequency ablation catheter may be accurately located to the lesion site through the location sensor, and an ablation needle can further accurately penetrate into the lesion tissue to perform radio frequency ablation therapy, so as to achieve targeted precise ablation therapy and reduce the damage to normal tissues.

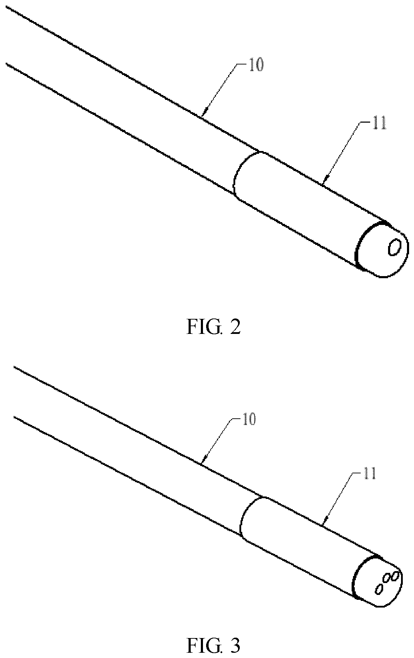

In various embodiments of the invention, the implementation structure of the catheter body 10 is not limited. Alternatively, the catheter body 10 may be a single-lumen structure, as shown in FIG. 2. The location sensor 12 and the ablation needle 13 are both provided within the lumen of the catheter body 10 and are both insulated. Alternatively, the catheter body 10 may be a multi-lumen structure, as shown in FIG. 3. The location sensor 12 is set in one of the lumens; and the ablation needle 13 is set in others of the lumens. The total number of the ablation needle 13 may be one or more. One or more ablation needles 13 may be provided in different lumens. Herein, the same number of ablation needles 13 may be provided in each lumen, i.e. the ablation needles 13 are evenly distributed in these lumens. Alternatively, a different number of ablation needles 13 may be provided within each lumen.

In various embodiments of the present application, the ablation needle 13 needs to have a certain elasticity and needs to be electrically conductive, so as to facilitate radiofrequency ablation. Alternatively, the entire ablation needle 13 may be electrically conductive. Alternatively, other than the needle tip portion, the ablation needle 13 are provided with an insulating layer for insulation treatment, so as to reduce electromagnetic interference.

In the various embodiments of the present application, the shape of the ablation needle 13 is not limited, and all shapes having a certain elasticity, capable of moving forward or backward inside and external to the catheter body 10 and being penetrated into the lesion tissue are suitable for the embodiments of the present application.

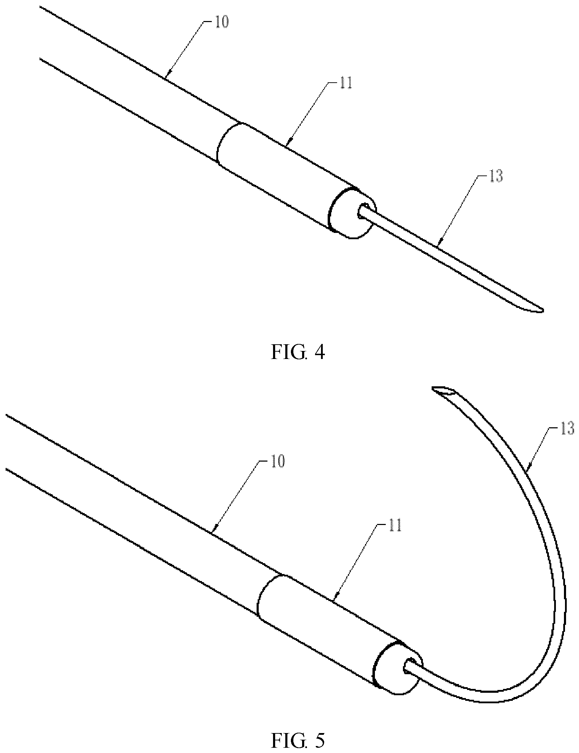

Alternatively, the ablation needle 13 may be a straight needle. The so-called straight needle is straight after pushing out of the head of the catheter body 10. For example, the straight needle may be a single elastic metal wire, or it may be a structure in which a plurality of metal wires are twisted or braided. When the straight needle adopts a structure in which a plurality of metal wires are twisted or braided, it may be a one-layer braided structure or a multi-layer braided structure. The portion of the straight needle within the catheter body 10 may curve as the catheter body 10 curves and may conduct the current. The straight needle may be connected to an external control system by the wires. Alternatively, the straight needle may be completely electrically conductive, or only the needle tip portion may be electrically conductive while other portions are insulated. Herein, a single metal wire or multiple metal wires are selected to fully utilize the force transmission of the metal wires, so that the straight needle can flexibly push out or retract of the catheter body 10. Further, the straight needle may be a single needle or a plurality of needles. The radiofrequency ablation catheter with a single straight needle is easier to operate, the puncture direction is easy to control, and the radiofrequency ablation catheter may be accurately punctured inside the lesion tissue., A radiofrequency ablation catheter having a single straight needle, in conjunction with the single-lumen structure of the catheter body 10, is shown in FIG. 4. Alternatively, the ablation needle 13 may be a curved needle. The so-called curved needle is curved, after pushing out of the head of the catheter body 10, at a set angle to the catheter body 10. The embodiments do not limit the curved shape of the curved needle and the curved needle may have a variety of sizes. Similar to the straight needle, the curved needle may be a single metal wire having elasticity, or it may be a structure in which a plurality of metal wires are twisted or braided. When the curved needle adopts a structure in which a plurality of metal wires are twisted or braided, it may be a one-layer braided structure or a multi-layer braided structure. The portion of the curved needle within the catheter body 10 may curve as the catheter body 10 curves and may conduct the current. The curved needle may be connected to an external control system by the wire. Alternatively, the curved needle may be completely electrically conductive, or only the needle tip portion may be conductive while other portions are insulated. The needle tip portion of the curved needle is curved, naturally returns to a curved shape after pushing out of the head of the catheter body 10, and may be inserted into the lesion tissue. Herein, the direction in which the tip portion is curved is relatively fixed to the head of the catheter body 10. The catheter body 10 may transmit torque and turning the catheter body 10 may change the orientation of the ablation needle 13. Herein, the actual direction of the curved needle may be accurately determined by means of the location sensor 12. Further, the curved needle may be a single needle or a plurality of needles. A radiofrequency ablation catheter with a single curved needle may be easily punctured into a relatively lateral lesion tissue (such as a tumor at one side of a bronchus) for ablation therapy, and the puncture direction can be easily controlled., A radiofrequency ablation catheter having a single curved needle, in conjunction with the single-lumen structure of the catheter body 10, is shown in FIG. 5.

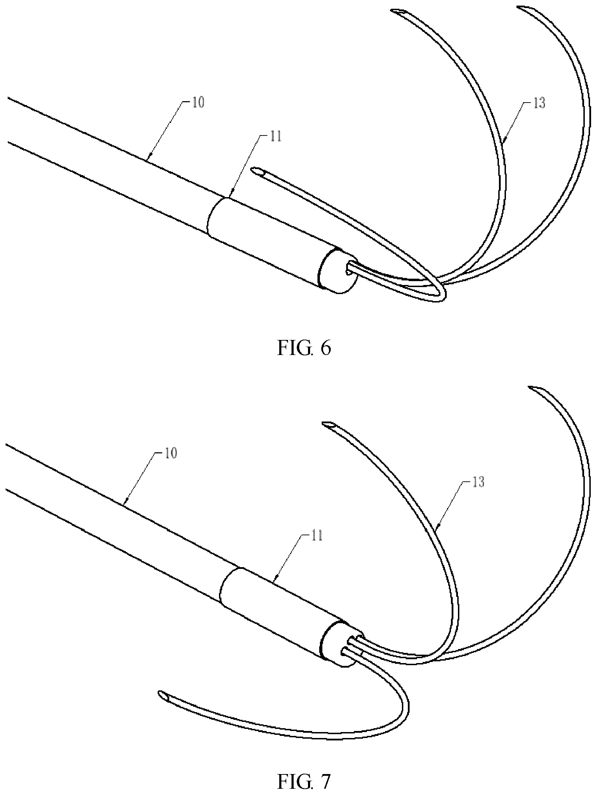

For a radiofrequency ablation catheter with a plurality of curved needles, the tail portions of the plurality of the curved needles are fixed together. The plurality of the curved needles are insulated from each other. The curved needles naturally return to a curved shape in multiple directions after pushing out of the head of the catheter body 10, and may be inserted into lesion tissues from different directions. The number of the curved needles is not limited in the embodiments, and may be, for example, two, three or more. In addition, the embodiments also do not limit the curved shape and the curved angle of the curved needle. The radiofrequency ablation catheter with a plurality of curved needles may be punctured into different portions of the lesion tissue, thereby increasing the contact area with the lesion tissue and obtaining a larger effective ablation area, which has a better ablation treatment effect on larger lesion tissue. A radiofrequency ablation catheter having a plurality of curved needles, in conjunction with the single-lumen structure of the catheter body 10, is shown in FIG. 6. A radiofrequency ablation catheter having a plurality of curved needles, in conjunction with the multi-lumen structure of the catheter body 10, is shown in FIG. 7.

In the process of performing radiofrequency ablation using the radiofrequency ablation catheter, it is desirable to control the pushing out or retraction of the ablation needle 13 from the head of the catheter body 10. Therefore, the radiofrequency ablation catheter of the embodiment may further include: means for controlling the pushing or retraction of the ablation needle 13 from the head of the catheter body 10. The means for controlling the pushing or retraction of the ablation needle 13 from the head of the catheter body 10 is not limited herein. Any structures or assemblies capable of controlling the pushing out of the ablation needle 13 from the head of the catheter body 10 is suitable for use in the embodiments of the present application.

In an alternative embodiment, the trailing end of the ablation needle 13 is connected to one end of a push-pull/torque transmission mechanism, and the other end of the push-pull/torque transmission mechanism is connected to a push-pull handle. The ablation needle 13 may be controlled to push out of the head of the catheter body 10 by pushing the push-pull handle inwards; the ablation needle 13 may be controlled to retract from the head of the catheter body 10 by pulling the push-pull handle outwardly. Alternatively, the push-pull/torque transmission mechanism may be a single steel wire rope, or a steel wire rope woven formed by a plurality of wires. Alternatively, the push-pull/torque transmission mechanism may also be a single super elastic nitinol (NiTi) wire, or a nickel-titanium rope twisted by a plurality of super elastic nitinol wires.

In an alternative embodiment, as shown in FIG. 1a, the ring electrode 11 is provided with a temperature sensor 14 therein; the temperature sensor 14 is connected to a control system external to the radiofrequency ablation catheter through a wire and is mainly used to test and control the temperature in the radiofrequency ablation process so as to ensure the curative effect of the radiofrequency ablation. For example, the temperature sensor 14 is mainly used to detect a temperature of the ring electrode 11 in real time and feeding back the temperature to an external control system so as to control the power and time of the current generated by the ring electrode 11, thereby achieving a curative purpose.

The type of the location sensor is not limited in various embodiments of the present application. Alternatively, the location sensor may be an electromagnetic navigation location sensor, but is not limited thereto. For example, a 5-degree-of-freedom electromagnetic navigation sensor or a 6-degree-of-freedom electromagnetic navigation sensor may be employed.

In actual use, radiofrequency ablation may interfere with the location sensor and affect the location effect, so a location sensor with an electromagnetic shielding layer, such as, but not limited to, a 5-degree-of-freedom electromagnetic navigation sensor, may be adopted. In addition, the wires connecting the location sensor to the external control system may also be shielded to further reduce interference during current signal transmission. By adopting the location sensor with the electromagnetic shielding layer, not only interference caused by radiofrequency ablation may be shielded and location accuracy is improved, but also real-time locationing may be performed in the radiofrequency ablation process to prevent ablation location deviation caused by unexpected movement of the ablation catheter and improve treatment accuracy and efficiency.

It is hereby noted that the main components of the radiofrequency ablation catheter are shown in the drawings attached to the present application, and some wires or connection wires are not shown. The ablation needle 13, the ring electrode 11, the location sensor 12, the temperature sensor 14 and the like are all required to be connected to the external control system by wires. Alternatively, these wires may be located within the lumens inside the catheter body 10 and connected to the external control system.

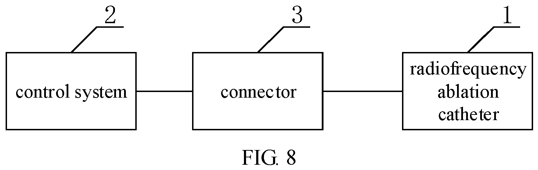

The radiofrequency ablation catheter provided by the above-mentioned embodiments of the present application may be applied to a radiofrequency ablation system, and may cooperate with other components in the radiofrequency ablation system to complete the radiofrequency ablation. FIG. 8 is a schematic diagram of a structure of a radiofrequency ablation system provided by another embodiment of the present application. As shown in FIG. 8, the radiofrequency ablation system includes: a radiofrequency ablation catheter 1, a control system 2 and a connector 3. The control system 2 is mainly used for controlling the operation of radiofrequency ablation catheter 1. The radiofrequency ablation catheter 1 is operated under the control of the control system 2. The radiofrequency ablation catheter 1 is connected with the control system 2 through the connector 3. Namely, the radiofrequency ablation catheter 1 is connected with an end of the connector 3, and the control system 2 is connected with the other end of the connector 3. The structure of the radiofrequency ablation catheter 1 is as shown in FIG. 1a to FIG. 5, so that no more details will be described here.

Taking the structure of the radiofrequency ablation catheter 1 shown in FIG. 1a as an example, the connection between the radiofrequency ablation catheter 1 and the connector 3 mainly refers to that: the ring electrode, the ablation needle and the location sensor are respectively connected with the connector 3. Herein, the ring electrode, the ablation needle and the location sensor may be connected with the connector 3 through lead wires. the implementation manner of the lead wires is not limited in the present embodiments. Preferably, these lead wires are connected with the connector 3 through the interior of the catheter body.

Preferably, the radiofrequency ablation catheter 1 is connected to the connector 3 in such a manner that: the ring electrode and the ablation needle are respectively connected with the connector 3 through one lead wire, and the location sensor is connected with the connector 3 through multiple lead wires, specifically depending on the type of the location sensor. For example, if the location sensor adopts a 5 degree-of-freedom electromagnetic navigation sensor, two lead wires may be adopted to connect the location sensor with the connector 3. If the location sensor adopts the 6 degree-of-freedom electromagnetic navigation sensor, four lead wires may be used to connect the location sensor with the connector 3. Optionally, if the ring electrode is provided with a temperature sensor therein, then the temperature sensor may be connected with the connector 3 through two lead wires.

A working principle of the radiofrequency ablation system is briefly described below by taking such a case that the radiofrequency ablation system performs the radiofrequency ablation on the basis of a CT (Computed Tomography) and/or a three-dimensional bronchus tree image of a lesion region (such as a lung region).

At an initial path planning stage:
the control system 2 imports CT image data and/or the three-dimensional bronchus tree graphics of the lesion region, and an initial navigation path via multiple levels of bronchus of the lesion region to the main trachea is drawn on the CT image data and/or the three-dimensional bronchus tree image.

At a path navigation stage:
the radiofrequency ablation catheter 1 is delivered to the airway through a bronchoscope along the initial navigation path, and a magnetic field generator in the control system 2 continuously generates magnetic fields. The location sensor in the radiofrequency ablation catheter 1 generates a current signal in response to the magnetic field in the airway and feeds the current signal back to the control system 2 in real time. The control system 2 continuously calculates position coordinates of the radiofrequency ablation catheter 1 according to the current signal fed back by the location sensor, and continuously guides the radiofrequency ablation catheter to advance along the navigation path in the bronchus tree until the radiofrequency ablation catheter 1 is guided to the lesion position; after the radiofrequency ablation catheter 1 reaches the intratracheal position, the control system 2 calculates the position and direction of the ablation needle in the radiofrequency ablation catheter 1 relative to the lesion tissue according to the current signal fed back by the location sensor.

At a radiofrequency ablation stage:
the ablation needle is pushed out of the catheter body of the radiofrequency ablation catheter 1 to enable the ablation needle to penetrate the lesion tissue, then the radiofrequency ablation generator in the control system 2 provides an ablation solution (mainly including output power, ablation time and the like of the ablation electrode) for the radiofrequency ablation catheter 1, so as to control the radiofrequency ablation catheter 1 to perform radiofrequency ablation. The radiofrequency ablation generator also may adjust the ablation solution according to treatment situation.

In addition, the control system 2 also may track the position coordinates of the radiofrequency ablation catheter 1 in real time according to the current signal fed back by the location sensor, and the position of the radiofrequency ablation catheter 1 in the airway is adjusted manually, so that the problem that the inaccurate position of the radiofrequency ablation catheter due to change of lesion positions caused by breath movement of the lung or other unexpected movements may be solved, and the radiofrequency ablation is performed more accurately.

In the radiofrequency ablation system provided in the embodiments, the radiofrequency ablation catheter is further provided with an ablation needle in addition to the ring electrode, and the ablation needle which is capable of moving forward or backward and pushed out of the head of the catheter body and penetrated into the lesion tissue; in addition, a location sensor is provided in the catheter body, and navigation can be performed with the location sensor, such that the radiofrequency ablation catheter may be accurately located to the lesion position, and an ablation needle can further accurately penetrate into the lesion tissue to perform radio frequency ablation therapy, so as to achieve targeted precise ablation therapy and reduce the damage to normal tissues.

Persons skilled in the art should understand that the embodiments of the invention may provide a method, a system or a computer program product. Therefore, the invention may adopt the form of a complete hardware embodiment, a complete software embodiment, or a software and hardware combined embodiment. In addition, the invention may adopt the form of a computer program product implemented on one or multiple computer available storage media (including, but not limited to, a magnetic disk memory, a Compact Disc Read-Only Memory (CD-ROM), an optical memory and the like) including computer-sensitive program codes.

The present invention is described by referring to flowcharts and/or block diagrams of methods, devices (systems) and computer program products according to the embodiments of the present invention. It should be understood that each flow and/or each block in the flowcharts and/or the block diagrams and a combination of the flows and/or the blocks in the flowcharts and/or the block diagrams may be implemented by the computer program instructions. These computer program instructions may be provided for a processor of a general computer, a dedicated computer, an embedded processor or other programmable data processing devices to generate a machine, so that an apparatus for achieving functions designated in one or more flows of the flowcharts and/or one or more blocks of the block diagrams is generated via instructions executed by the processor of the computers or the other programmable data processing devices.

These computer program instructions also may be stored in a computer readable memory capable of guiding the computer or other programmable data processing devices to work in a specific manner, so that a manufactured product including an instruction apparatus is generated via the instructions stored in the computer-readable memory, and the instruction apparatus achieves the functions designated in one or multiple flow of flowcharts and/or one or multiple blocks of the block diagrams.

These computer program instructions, which can also be loaded onto the computers or the other programmable data processing devices, enable the computers to implement a series of operation steps on the computers or the other programmable data processing devices; therefore, the instructions executed on the computers or the other programmable data processing devices provide steps of achieving the functions designated in one or multiple flows of the flowcharts and/or one or multiple blocks of the block diagrams.

In a typical configuration, a computing device includes one or multiple Central Processing Units (CPUs), one or multiple input/output interfaces, one or multiple network interfaces and one or multiple internal memories.

The memory may include a non-persistent memory, a Random Access Memory (RAM) and/or a non-volatile memory, etc., such as an ROM or a flash RAM, in a computer-readable medium. The memory is an example of the computer-readable medium.

The computer-readable medium includes persistent, non-persistent, removable media and non-removable media which may realize information storage by any methods or technologies. Information may be computer readable instructions, data structures, modules of programs or other data. Examples of computer storage media include, but are not limited to, a Phase-change Random Access Memory (PRAM), a Static Random Access Memory (SRAM), a Dynamic Random Access Memory (DRAM), other types of RAMs, an ROM, an Electrically Erasable Programmable Read-Only Memory (EEPROM), a flash memory or other memory technologies, a CD-ROM, a Digital Video Disk (DVD) or other optical memories, a magnetic cartridge type magnetic tape, a magnetic tape/disk storage device or other magnetic storage devices or any other non-transmission media, and may be used for storing information accessible by the computing device. The computer readable medium does not include transitory media, such as modulated data signals and carriers, according to definitions herein.

It also should be noted that terms "include", "comprise" or any other variants are meant to cover non-exclusive inclusions, so that a process, method, commodity or device that includes a series of elements not only includes those elements, but also includes other elements which are not definitely listed, or further includes inherent elements of this process, method, commodity or device. Without more restrictions, elements defined by a sentence "includes a/an ..." do not exclude that the process, method, commodity or device that includes the elements still includes other identical elements.

Persons skilled in the art should understand that the embodiments of the present application may provide a method, a system or a computer program product. Therefore, the present application may adopt the form of a complete hardware embodiment, a complete software embodiment, or a software and hardware combined embodiment. In addition, the present application may adopt the form of a computer program product implemented on one or multiple computer-sensitive storage media (including, but not limited to, a magnetic disk memory, a CD-ROM, an optical memory and the like) including computer-sensitive program codes.

The above are merely the embodiments of the present application, but are not intended to limit the present application. Persons skilled in the art can make various changes and modifications to the present application. Any modifications, equivalent replacements, improvements and the like that are made without departing from the spirit and the principle of the present application shall fall within the protection scope defined by the appended claims of the present application.

## Claims

1. A radiofrequency ablation catheter, comprising:
a catheter body;
a ring electrode, annularly provided at a head of the catheter body;
a location sensor, provided in the catheter body; and
an ablation needle, provided in the catheter body and capable of moving forward or backward,
wherein the ablation needle, when applied, pushes out of the head of the catheter body and
penetrates into a lesion tissue.

2. The radiofrequency ablation catheter as claimed in claim 1, wherein the location sensor is configured to output, according to a location magnetic field in a space where the location sensor is located, a current signal to a control system external to the radiofrequency ablation catheter, such that the control system determines a position of the radiofrequency ablation catheter and controls the ablation needle and the ring electrode to perform radiofrequency ablation.

3. The radiofrequency ablation catheter as claimed in claim 1, wherein the catheter body has a multi-lumen structure; the location sensor is set in one of lumens and the ablation needle is set in others of the lumens.

4. The radiofrequency ablation catheter as claimed in claim 3, wherein the number of ablation needles provided in each of the other lumens is the same.

5. The radiofrequency ablation catheter as claimed in claim 1, wherein the ablation needle is a straight needle which is straight after pushing out of the head of the catheter body; or,
the ablation needle is a curved needle which is curving, after pushing out of the head of the catheter body, at a set angle with respect to the catheter body.

6. The radiofrequency ablation catheter as claimed in claim 1, wherein a tail end of the ablation needle is connected to one end of a push-pull/torque transmission mechanism, and another end of the push-pull/torque transmission mechanism is connected to a push-pull handle.

7. The radiofrequency ablation catheter as claimed in claim 1, wherein the ring electrode is provided with a temperature sensor therein.

8. The radiofrequency ablation catheter as claimed in claim 1, wherein electrodes of at least two polarities are present in the ablation needle and the ring electrode.

9. The radiofrequency ablation catheter as claimed in claim 8, wherein a polarity of the ablation needle is opposite to a polarity of the ring electrode.

10. The radiofrequency ablation catheter as claimed in claim 1, wherein a portion, other than the needle tip, of the ablation needle is provided with an insulating layer.

11. A radiofrequency ablation system, comprising: the radiofrequency ablation catheter as claimed in any one of claims 1-10, a control system for controlling operations of the radiofrequency ablation catheter, and a connector for connecting the radiofrequency ablation catheter with the control system.
